# EUROPEAN PATENT APPLICATION

(11) **EP 2 067 478 A1**
(43) Date of publication of application: **10.06.2009**
(21) Application number: 07806812.9
(22) Date of filing: 06.09.2007
(51) Int. Cl.: A61K 33/00, A61K 33/24, A61K 35/56, A61P 3/10

(54) **AGENT FOR PREVENTION OR TREATMENT OF BLOOD GLUCOSE LEVEL ELEVATION**

(30) Priority: 07.09.2006 JP 2006242951
(71) Applicant: University of The Ryukyus, Nakagami-gun, Okinawa 903-0213 (JP); Coral Biotech Kabushiki Kaisha, Naha-shi Okinawa 900-0002 (JP)
(72) Inventor: MAEHIRA, Fusako, Naha-shi, Okinawa 900-0011 (JP)
(74) Representative: Blodig, Wolfgang
(86) International application number: PCT/JP2007/067371
(87) International publication number: WO 2008/029869

(57) **Abstract**

An object is to find a natural material having an activity of preventing the elevation of blood glucose level and to provide a means which is able to prevent or treat the diabetes and there is provided an agent for prevention or treatment of the elevated blood glucose level where a compound containing soluble silica and/or strontium is an active ingredient.

SEQUENCE LISTING

<110>  University of the Ryukyus
       CORAL BIOTECH KK

<120>  Prophylatic/therapeutic agent for elevation of blood-glucose

<130>  PF-070012-WO

<150>  JP 2006-242951
<151>  2006-09-07

<160>  8

<170>  PatentIn version 3.4

<210>  1
<211>  24
<212>  DNA
<213>  Artificial

<220>
<223>  PCR primer

<400>  1
cgagccctgg caaagcattt gtat                                              24


<210>  2
<211>  24
<212>  DNA
<213>  Artificial

<220>
<223>  PCR primer

<400>  2
tgtctttcct gtcaagatcg ccct                                              24


<210>  3
<211>  24
<212>  DNA
<213>  Artificial

<220>
<223>  PCR primer

<400>  3
gcactggcaa gttctactgc aaca                                              24


<210>  4
<211>  24
<212>  DNA
<213>  Artificial

<220>
<223>  PCR primer

<400>  4
agagaacggc cttgtccttc ttga                                              24


<210>  5
<211>  24
<212>  DNA
<213>  Artificial

<220>
<223>  PCR primer

<400>  5
aagaacctga ggaagccgtt ctgt                                              24

<210>  6
<211>  24
<212>  DNA
<213>  Artificial

<220>
<223>  PCR primer

<400>  6
gcagccacaa acagggaaat gtca                                              24


<210>  7
<211>  24
<212>  DNA
<213>  Artificial

<220>
<223>  PCR primer

<400>  7
aaaggctctt tacctggtgt gtgg                                              24


<210>  8
<211>  24
<212>  DNA
<213>  Artificial

<220>
<223>  PCR primer

<400>  8
actgatccac aatgccacgc ttct                                              24

## Description

### Technical Field

The present invention relates to an agent for prevention or treatment of the elevated blood glucose level and, more particularly, it relates to an agent for prevention or treatment of the elevated blood glucose level in which an inorganic component is an active ingredient.

### Background Art

Obesity is increasing now being caused by a lifestyle of people of today such as high-fat meals or lack of exercise. There will be a hereditary factor and an environmental factor for the obesity and it has been said to be related to a mechanism of energy accumulation by way of precaution against hunger which is a hereditary factor for humans. Thus, when an excessive energy was ingested under the circumstance where energy supply from food was not always secured, it was advantageous for survival and prosperity of offspring to store the surplus thereof as neutral fat and to quickly release upon hunger or the like. However, in the present age with plenty food, the good energy efficiency as such rather backfires resulting in accumulation of fat and the obesity followed by onset of insulin-resistant type II diabetes.

It has been also known that, in a patient who has a hereditary factor of lowering in insulin secretion as such or, in other words, where resistance to insulin is low, diseases such as obesity, impaired glucose tolerance, hyperglycemia or hypertension are onset where the above is a common basis and that risk factors for arteriosclerosis increase.

Due to the above lifestyle such as high-fat meals or lack of exercise, number of patients suffering from diabetes in this country is presumed to be not less than 7,000,000 at present and the lifestyle as such is not able to be changed so easily whereby further increase in the diabetic patients and prediabetics is now a matter of concern.

### Disclosure of the Invention

### Problems that the Invention is to Solve

An object of the present invention is to decrease patients of diabetes or prediabetes which is a lifestyle-related disease from the view of primary prevention by means of foods, and its theme is to find a natural material having a suppressive action on the elevated blood glucose level and to provide a means which is able to prevent and treat the diabetes.

### Means for Solving the Problems

The present inventor has carried out intensive studies for solving the above problems and has found that a compound having a specific inorganic element is able to effectively suppress an elevation of blood glucose level and useful for prevention or treatment of diabetes whereupon the present invention has been achieved.

Thus, the present invention is an agent for prevention or treatment of the elevated blood glucose level where a compound containing soluble silicon and/or strontium is an active ingredient.

### Advantages of the Invention

When the agent for prevention or treatment of the elevated blood glucose level according to the present invention is given for a defined period, it lowers blood glucose level, blood insulin level, blood leptin level and the like, which are elevated in insulin-resistant type II diabetes.

Accordingly, the agent for prevention or treatment of the elevated blood glucose level according to the present invention is able to be easily administered or ingested as a drug, a health food and the like, and is able to be effectively utilized for diabetes which is a lifestyle disease and also for prevention of obesity and so forth.

### Best Mode for Carrying Out the Invention

A compound which contains soluble silicon and/or strontium used in the present invention means a compound which contains one of soluble silicon and strontium and a compound which contains both of soluble silicon and strontium.

Among them, examples of the compound which contains one of soluble silicon and strontium include a compound which contains soluble silicon and inorganic and organic compounds which contain strontium.

Examples of the compound which contains soluble silicon include metasilicic acid and silicic acid as well as sodium, potassium, calcium and magnesium salts thereof.

Examples of the inorganic compound which contains strontium include strontium chloride, strontium bromate, strontium bromide, strontium hydroxide, strontium oxide, strontium carbonate, strontium phosphate and strontium sulfate, while examples of the organic compound which contains strontium include strontium acetate, strontium lactate and strontium oxalate as well as a strontium-containing organic synthetic compound such as strontium ranelate (5-[bis(carboxy-methyl)-amino]-2-carboxy-4-cyano-3-thiophenacetic acid distrontium salt; S12911-PROTOS, Institut de Recherches Internationales Servier, Courbevoie, France).

Examples of the compound which contains both of soluble silicon and strontium include unburned coral calcium.

Among the above, unburned coral calcium which is a compound containing both of soluble silicon and strontium is an unburned product of FUUKA ZOSHO SANGO (the so-called weathered reef-building coral different from fossil stony coral) and is used as fine powder such as powder of an average particle size of about 10 to 5 µm. When the FUUKA ZOSHO SANGO is burned, soluble silicon disappears by burning whereby an effect of prevention, treatment and the like of diabetes is unable to be achieved.

Powder of unburned FUUKA ZOSHO SANGO (hereinafter referred to as "the ZOSHO SANGO powder") is prepared by pulverization of FUUKA ZOSHO SANGO collected in an area where coral reef is present such as Okinawa Prefecture and, as a composition, it contains 30 to 39% by mass (hereinafter, it will be simply mentioned as "%") of calcium (Ca) and 1.5 to 2.5% of magnesium (Mg) where the ratio by weight of Ca to Mg is from about 26:1 to 12:1. It also contains a small amount such as about 0.01 to 0.05% of silicon and 0.0005 to 0.002% thereof is contained as soluble silicon (cell membrane-permeating type or bioavailable type).

Since the ZOSHO SANGO powder is commercially available from, for example, Coral Biotech KK as "Coral Bio PW", it may be used. An example of analysis of the ZOSHO SANGO powder which is the Coral Bio PW is shown below.

| | |
|---|---|
| Calcium | 36.1% |
| Magnesium | 2.03% |
| Sulfur | 0.40% |
| Sodium | 0.32% |
| Strontium | 0.28% |
| Fluorine | 0.075% |
| Iron | 0.039% |
| Silicon | 0.025% |

An agent for prevention or treatment of the elevated blood glucose level according to the present invention is able to be prepared, for example, in such a manner that the compound containing soluble silicon and/or strontium is dispersed/dissolved in an appropriate solvent or is pulverized and then it is used as an active ingredient.

In that case, although a content of the soluble silicon which is an active ingredient in the agent for prevention or treatment of the elevated blood glucose level of the present invention is not particularly limited so far as it is within a range of showing no toxicity, its dose for an adult per day is preferably about 5 µg to 200 mg or, particularly preferably, 10 to 20 mg in case the soluble silicon is used solely.

With regard to strontium which is another active ingredient, although there is no particular limitation as well so far as it is within a range of showing no toxicity, its dose for an adult per day is preferably about 1.5 mg to 1,500 mg or, particularly preferably, 500 to 700 mg in case it is solely compounded. When a compound containing both soluble silicon and strontium is used, the contents thereof may be decided in accordance with the above.

As to one of the forms of the agent for prevention or treatment of the elevated blood glucose level according to the present invention, a pharmaceutical dosage form such as powder, granules, tablets or capsules may be exemplified. In making into such a form, various kinds of commonly used pharmaceutical carriers may be used if necessary. In the case of the pharmaceutical dosage form as such, it may be in a form by which the above dose is ingested once daily or ingested by dividing into several times a day.

In addition to the compound which contains soluble silicon and/or strontium, other health food materials such as various kinds of food additives, vitamins or minerals may be further compounded therewith so as to prepare an agent for prevention or treatment of the elevated blood glucose level of a health food type containing plural active ingredients.

It is also possible to make the agent for prevention or treatment of the elevated blood glucose level of the present invention into a food-additive type and to prepare a food/beverage having an action of prevention of the elevated blood glucose level by addition of various kinds of food materials thereto. Examples of the food/beverage as such include beverage such as refreshing beverage, nutritive beverage, fruit beverage or lactic acid bacterium beverage (including liquid concentrate and/or prepared powder for preparing such a beverage); frozen dessert such as ice cream or sherbet; processed product of cereals such as buckwheat noodles, *udon* noodles, bread, *mochi* (rice cake), various types of cooked rice meals, various types of pastas or dough skin of gyoza (Chinese-style dumpling); confectionery such as *ame* (candy with a starch base), candy, chocolate, snack food, biscuit, cookie, cracker, jelly or jam; processed fishery and livestock product such as *kamaboko* (steamed fish paste), *hanpen* (steamed cake of fish with starch), ham or sausage; milk product such as processed milk, cheese or butter; fat/oil and processed fat/oil food such as margarine, lard or mayonnaise; seasoning such as soy sauce, Worcester sauce, *miso* (fermented soybean paste), *ponzu* (sour orange-based sauce), sea tangle stock or soup stock; various kinds of ready-prepared food; pickles; and various forms of other health and nutritional (supplementary) food.

In the case of an agent for prevention or treatment of the elevated blood glucose level of a food additive type, an amount by which the final ingesting dose is the same as that mentioned above or below may be added to the food as well.

Although the action mechanism of the present invention is ambiguous in some respects, it is presumed to be as follows. Namely, a peroxisome proliferator-activated receptor (PPAR) γ is a target being activated by a thiazolidinedione derivative which is an antidiabetic agent as a ligand (Okuno A, et al., J. Clin. Invest., 101, 1354-1361 (1998)) and it has been said that the antidiabetic agent shows unfavorable effects on bone metabolism of patients in a long-term medical treatment.

Recently, there has been a report where gene polymorphism which lowers human PPAR-γ activity suppresses resistance to insulin or onset of diabetes (Hara K, et al., Biochem. Biophys. Res. Comnun., 271, 212-216 (2000)) or a report where, in PPAR-γ hetero-deficient mice which express about 50% of PPAR-γ of wild mice, obesity, resistance to insulin and onset of diabetes caused by a load with a high-fat food or a high-carbohydrate food are suppressed as compared with those of a wild type (Yamauchi T, et al., J. Biol. Chem., 276, 41245-41254 (2001)). Thus, a medium-degree suppression of fat cell PPAR-γ has anti-obesity and antidiabetic actions and possibility of treatment of type II diabetes by a PPAR-γ antagonist is proposed by Kadowaki, et al.

Soluble silicon or strontium used as an active ingredient in the agent for prevention and treatment of the elevated blood glucose level according to the present invention is thought to have such a lowering action for expression of the PPAR-γ to a medium extent and it is understood that, due to such an action, an antidiabetic effect is able to be achieved.

### Examples

The present invention will now be further illustrated by way of the following Examples but the present invention is not limited by those Examples at all.

### Example 1

Biochemical test:
Firstly, four kinds of feeds were prepared for animal tests. They are (1) a feed (CT feed) where calcium carbonate was added to a Ca-deficient pure feed (manufactured by Oriental Yeast Co., Ltd.) so as to make the calcium content 1%, (2) a feed (CS feed) where unburned coral calcium (Coral Bio PW; manufactured by Coral Biotech KK) was added to the Ca-deficient pure feed so as to make the calcium content 1%, (3) a feed (Si feed) where sodium metasilicate was added to the above (1) so as to make the silicon amount 50 ppm and (4) a feed (Sr feed) where strontium chloride was added to the above (1) so as to make the strontium amount 750 ppm.

Male spontaneously obese insulin-resistant diabetic mice (KKAy) (five weeks age) were made into groups where each group comprised eight mice and fed for two months with the above four feeds (each group was called control group, CS group, Si group and Sr group). Feeding of the mice of those four groups was conducted in a single feeding manner and water was freely taken by each mouse.

Blood glucose level during the feeding period (until the 51st day) and blood glucose level, blood insulin concentration, blood leptin concentration and adiponectin concentration were measured when the feeding finished (on the 56th day) whereby the action of each ingredient on the mice was checked. Further, in order to compare with the diabetic model mice, normal mice (eight males) of the same 13 weeks age were used as a normal group and their blood was collected upon finishing the feeding followed by measuring each of the above concentrations.

### (Measurement of blood glucose level)

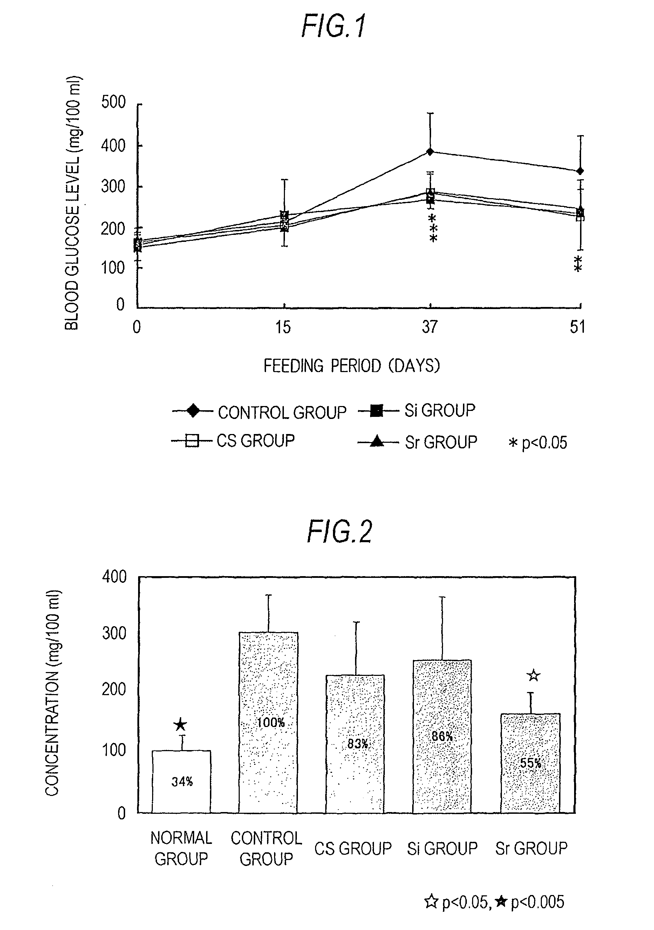

During the feeding period for the two months, blood was collected from tail vein of the mice upon the initiation of the feeding (on the 0th day) and on the 15th, 37th and 51st days from the initiation of the feeding and the blood glucose level therein was measured. The result is shown in Fig. 1.

As will be apparent from Fig. 1, all of mean values of the CS group (163, 206, 285 and 227 mg/dl), mean values of the Si group (156, 231, 268 and 235 mg/dl) and mean values of the Sr group (150, 200, 288 and 247 mg/dl) showed low values as compared with the changes in the mean values of the control group (167, 214, 386 and 339 mg/dl) and, particularly on the 37th day and thereafter, all of the test groups were about 70% of the blood glucose level of the control group whereby the blood glucose level elevation was significantly suppressed.

Further, as shown in Fig. 2, the blood glucose levels upon finishing the feeding (on the 56th day) were 83% for CS group, 86% for Si group and 55% for Sr group and were clearly low as compared with 306 mg/dl (100%) of the control group whereby it was shown that the agent for prevention or treatment of the elevated blood glucose level according to the present invention brought down the blood glucose level to the level of the normal mice (34%).

### (Measurement of plasma insulin concentration)

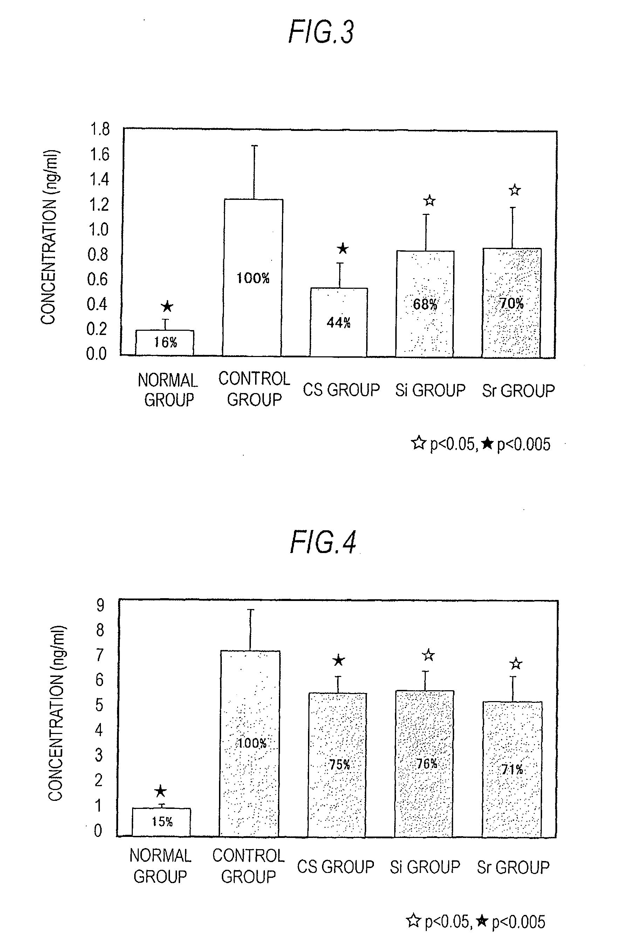

Concentration of insulin in plasma was measured upon finishing the feeding (on the 56th day). The result is shown in Fig. 3.

As will be apparent from Fig. 3, the plasma insulin concentration clearly lowered to 44% for CS group, 68% for Si group and 70% for Sr group as compared with 7.0 ng/ml (100%) of the control group whereby it was shown that the agent for prevention or treatment of the elevated blood glucose level according to the present invention brought down the resistance to insulin to the level of the normal mice (16%).

### (Measurement of plasma leptin concentration)

Leptin in plasma which is synthesized in adipocytes and discharged into blood and had a positive correlation to the body fat promotes the carbohydrate metabolic turnover and the carbohydrate uptake mostly via a central nerve system and acts as an antidiabetic factor. Concentration of leptin was also measured upon finishing the feeding (on the 56th day). The result is shown in Fig. 4.

As will be apparent from Fig. 4, the plasma leptin concentration significantly lowered to 75% for CS group, 76% for Si group and 71% for Sr group as compared with 7.3 ng/ml (100%) of the control group whereby it was shown that the agent for prevention or treatment of the elevated blood glucose level according to the present invention brought down the plasma leptin concentration to the level of the normal mice (15%) as a result of reduction in the body fat (improvement in obesity) or that, the same as in insulin, it improved the resistance to leptin and reduced the blood glucose level.

### (Measurement of plasma adiponectin concentration)

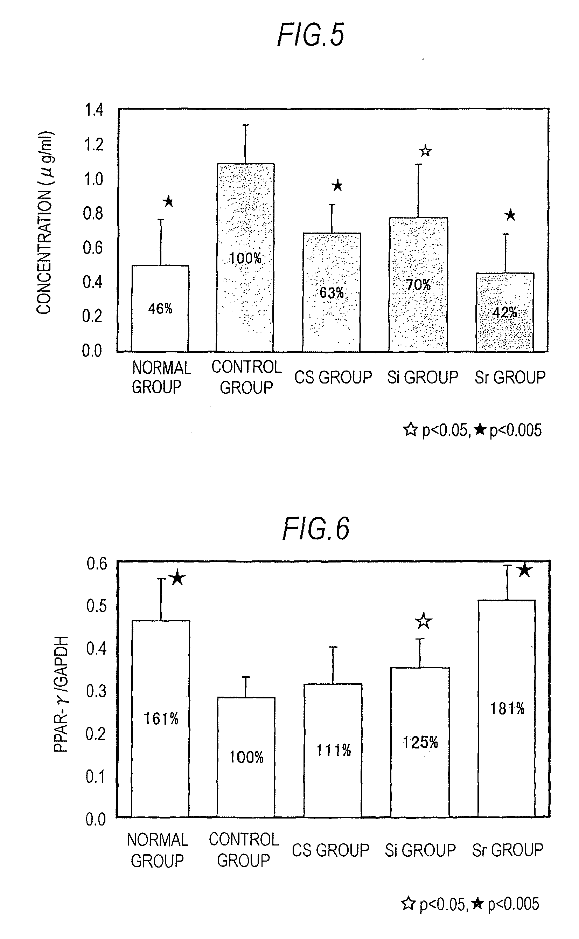

It has been said that a plasma adiponectin which is an anti-antidiabetic and anti-arteriosclerotic factor derived from fat tissues enhances insulin sensitivity and improves insulin resistant diabetes due to its action of activating the fatty acid oxidation in systemic muscles and reducing the neutral fat amount (Iichiro Shimomura, Toru Funahashi and Yuji Matsuzawa: Adiponectin - Anti-antidiabetic and anti-arteriosclerotic factor derived from fat tissues, Molecular Medicine, Vol. 39, No. 4, 416-423, 2002). Like insulin and leptin, this substance achieves its action via a specific receptor (Toshimasa Yamauchi and Takashi Kadowaki: Adiponectin Receptor, Molecular Medicine, Vol. 42, No. 1, 22-29, 2005) and, therefore, it has been said to cause such a vicious cycle that a high level of insulin in blood in the case of insulin resistance lowers the expression of specific receptor of skeleton muscle and liver, induces the insufficient adiponectin action, results in adiponectic resistance in obesity and insulin resistant diabetes is promoted. The concentration of adiponectin was also measured upon finishing the feeding (on the 56th day). The result is shown in Fig. 5.

As will be apparent from Fig. 5, the plasma adiponectin concentration significantly lowered to 63% for CS group, 70% for Si group and 42% for Sr group as compared with 1.1 µg/ml (100%) for the control group whereby it is shown that the agent for prevention or treatment of the elevated blood glucose level according to the present invention lowers the plasma adiponectin concentration to the level of normal mice (46%), the same as in the case of insulin and leptin, improving the adiponectic resistance and lowering the blood glucose level.

### (Safety)

No toxicity was noted in the result where the mice of Si group and CS group were subjected to a long-term feeding for six months under the above feeding condition.

### Example 2

Genetic examination (1):
The mice in each group of Example 1 were sacrificed. According to the conventional method, RNA was extracted from the cells of pancreas and kidney. cDNA was prepared by a reverse transcription reaction from the RNA and quantitative determination of expression amount of mRNA related to diabetes was carried out by means of a real-time quantitative PCR. In the following experiments, quantitative determination of the expression amount of mRNA was analyzed using Mx 3000P (manufactured by Stratagene). In all cases, condition for the PCR was that a hot start was conduced at 95°C for 10 minutes and then a cycle comprising 95°C for 30 seconds, 60°C for 1 minute and 72°C for 30 seconds was repeated for 45 times. At the same time, dilution rows of five stages were prepared using a control RNA of 5 ng/µl concentration and expression amount of RNA was determined from the resulting standard curve. As to a reagent for the PCR, Brilliant SYBR Green QPCR Master Mix (manufactured by Stratagene) was used in accordance with a protocol for its manual for use. A primer used for each test is described below.
Incidentally, expression amount of mRNA of a test substance in each test was indicated in terms of a ng ratio of the commonly expressed GAPDH enzyme to the amount of mRNA (internal standard).

### (Quantification of expression of PPAR-γ mRNA in pancreas)

Expression amount of mRNA of PPAR-γ promoting the gene transcription of a glucose-sensing apparatus of the pancreatic β-cells where insulin is synthesized and secreted is decreased in diabetes and a high blood glucose level in pancreas caused thereby induces the stimulation of an excessive insulin secretion resulting in one of the causes for insulin resistance (Kim HI, Ahn YH: Role of peroxisome proliferators-activated receptor-γ in the glucose-sensing apparatus of liver and β-cells. Diabetes, 53 (Suppl. 1), S60-S65 (2004)).

The expression amount of PPAR-γ mRNA was measured by the PCR using the following primer. The result is shown in Fig. 6.
PPAR-γ:
   (Forward: SEQ ID NO: 1)
      5'-CGAGCCCTGGCAAAGCATTTGTAT-3'
   (Reverse: SEQ ID NO: 2)
      5'-TGTCTTTCCTGTCAAGATCGCCCT-3'

As will be apparent from Fig. 6, the expression amount of mRNA of PPAR-γ significantly elevated to 111% for CS group, 125% for Si group and 181% for Sr group as compared with 0.28 (100%) for the control group whereby it was shown that the agent for prevention and treatment of the elevated blood glucose level according to the present invention raised the plasma adiponectin concentration to the level of the normal mice (161%), the same as in the case of insulin and leptin, improving the glucose sensitivity or insulin resistance and lowering the blood glucose level.

### (Quantification of expression of adiponectin mRNA in pancreas)

It has been reported that insulin resistance is released by adiponectin via promotion of the intra-tissue fatty acid combustion (Yamauchi T, Kamon J, Waki H, et al. The fat-derived hormone adiponectin reverses insulin resistance associated with both lipoatrophy and obesity. Nat. Med., 7, 941-946 (2001)). Among the clinically used pharmaceuticals at present, one which clearly increases the adiponectin in blood is only a thiazolidine derivative which is a PPAR-γ ligand (Maeda N, Takahashi M, Funahashi T, et al., PPARγ ligands increase expression and plasma concentration of adiponectin, an adipose-derived protein. Diabetes, 50, 2094-2099 (2001)) and it has been reported that the elevation of expression of adiponectin is conducted via PPAR-γ (Iwaki M, Matsuda M, Maeda N, et al., Induction of adiponectin, a fat-derived antidiabetic and antiathrogenic factor, by nuclear receptors. Diabetes, 52, 1655-1663 (2003)).

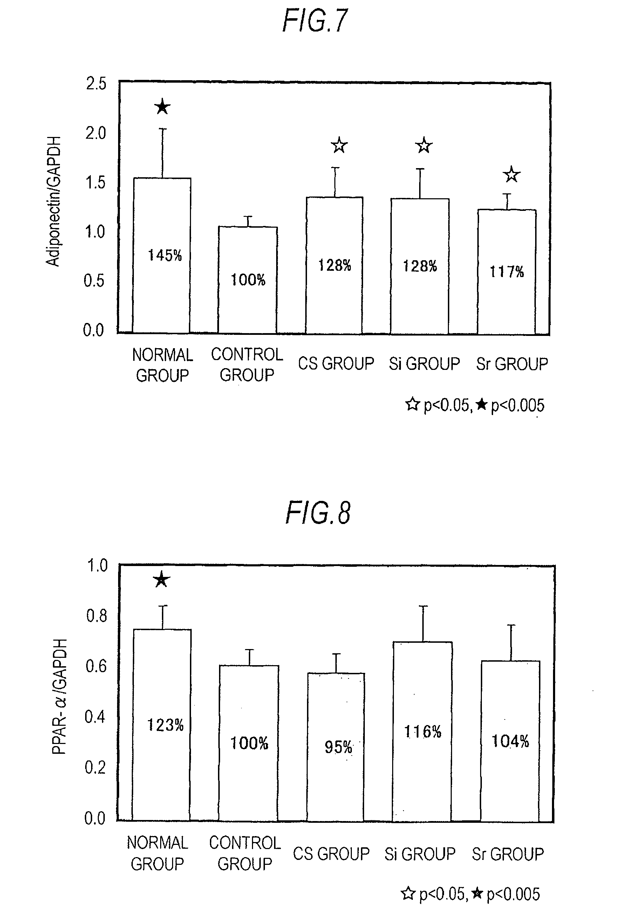

Expression amount of adiponectin mRNA was measured by the PCR using the following primers. The result is shown in Fig. 7.
Adiponectin:
   (Forward: SEQ ID NO: 3)
      5'-GCACTGGCAAGTTCTACTGCAACA-3'
   (Reverse: SEQ ID No: 4)
      5'-AGAGAACGGCCTTGTCCTTCTTGA-3'

As will be apparent from Fig. 7, an expression amount of adiponectin mRNA significantly elevated to 128% for CS group, 128% for Si group and 117% for Sr group as compared with 1.07 (100%) for the control group and it was shown that the agent for prevention and treatment of the elevated blood glucose level according to the present invention raised the expression amount of pancreatic adiponectin to the level of the normal mice (145%) via a significant rise of the above expression amount of PPAR-γ m-RNA, improving the glucose sensitivity or insulin resistance and lowering the blood glucose level.

### (Quantification of expression of PPAR-α mRNA in pancreas)

An expression amount of PPAR-α mRNA is suppressed by a high glucose concentration, promoting the insulin secretion from β-cells and participating in the adjustment of insulin secretion by glucose stimulation, whereas high fatty acid concentration raises its expression amount whereby promotes oxidative decomposition of fatty acid and intracellular transport of lipid (Sugden MC, Holness MJ: Potential role of peroxisome proliferators-activated receptor-α in the glucose-stimulated insulin secretion. Diabetes, 53 (Suppl. 1): S71-S81, 2004). It has been clarified that the mechanism for a promotion action of the fatty acid combustion by adiponectin is via an increase in the expression amount of PPAR-α m-RNA by adiponectin (Yamauchi T, Kamon J, Waki H, et al., The fat-derived hormone adiponectin reverses insulin resistance associated with both lipoatrophy and obesity. Nat. Med. 7, 941-946 (2001)).

The expression amount of the PPAR-α mRNA was measured by the PCR using the following primers. The result is shown in Fig. 8.
PPAR-α:
   (Forward: SEQ ID NO: 5)
      5'-AAGAACCTGAGGAAGCCGTTCTGT-3'
   (Reverse: SEQ ID NO: 6)
      5'-GCAGCCACAAACAGGGAAATGTCA-3'

As will be apparent from Fig. 8, little change was noted such as that 95% for CS group, 116% for Si group and 104% for Sr group as compared with 0.61 (100%) for the control group in which value was about 80% suppression of the normal mice level (123%). From this result, it is shown that the agent for prevention or treatment of the elevated blood glucose level according to the present invention does not bring a high fatty acid concentration.

As shown in Figs. 1 and 2 for Example 1, the agent for prevention or treatment of the elevated blood glucose level according to the present invention reduced a blood glucose level by about 30% of about 300 mg/dl, but the blood glucose level still remained about 200 mg/dl so that a suppression of a rise in expression of PPAR-α mRNA by glucose is presumed. As mentioned before, since significant rises in the expression amount of adiponectin which increases the expression amount of PPAR-α mRNA and further in that of PPAR-γ mRNA which promotes the expression of the adiponectin were observed, it is likely to be on the way to the normalization of blood glucose level.

### (Quantification of expression of insulin mRNA in pancreas)

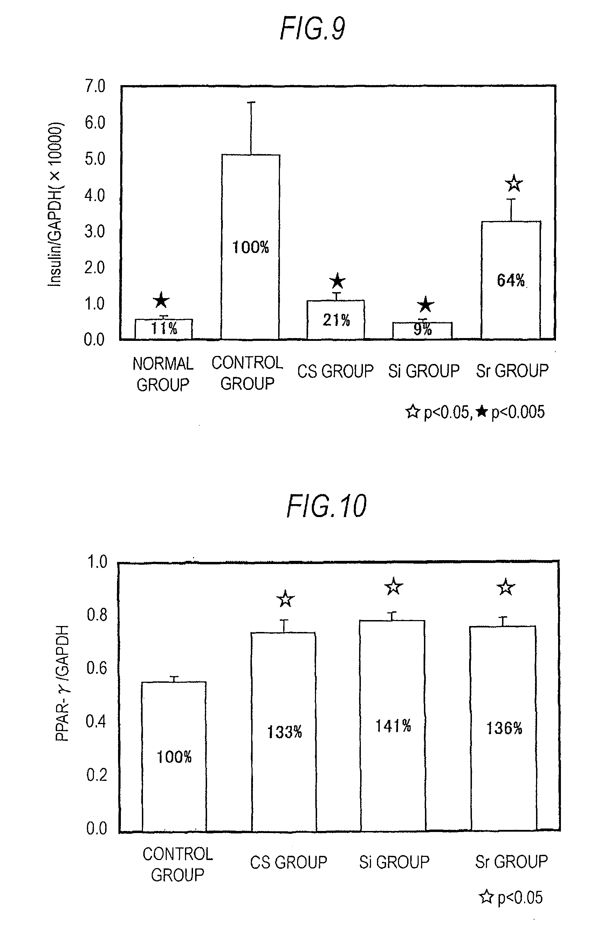

Expression amount of insulin mRNA in the pancreas was measured by the PCR using the following primers. The result is shown in Fig. 9.
Insulin:
   (Forward: SEQ ID NO: 7)
      5'-AAAGGCTCTTTACCTGGTGTGTGG-3'
   (Reverse: SEQ ID NO: 8)
      5'-ACTGATCCACAATGCCACGCTTCT-3'

As will be apparent from Fig. 9, while in the case of a normal mice, expression amount of insulin mRNA from pancreas which is a sole organ synthesizing and secreting the insulin is in a considerable amount (5.5 × 10³), in the control group of the obese insulin resistant diabetic mice, insulin in an amount of 10-fold (5.12 × 10⁴ (100%)) was synthesized and secreted. On the contrary, in CS group, Si group and Sr group, significant decrease as low as 21%, 9% and 64%, respectively, of the control group was noted whereby it is shown that the agent for prevention or treatment of the elevated blood glucose level according to the present invention lowered the expression amount of insulin mRNA of pancreas being reflected to blood insulin concentration to the level of normal mice (11%), thus improving the insulin resistance and lowering the blood glucose level.

### (Quantification of expression of PPAR-γ mRNA in kidney)

When diabetic nephropathy leads to nephritic syndrome, production of VLDL in liver increases corresponding to the loss of albumin into urine whereby neutral fat and cholesterol increase. Hyperglycemia is a risk factor for progress of glomerular dysfunction and fat disposal in renal tissues is believed to be important for prevention of the progress of nephropathy.

Expression amount of PPAR-γ mRNA in kidney which adjusts adiponectin and its expression promoting the fatty acid combustion in tissues thereof was measured by the PCR using the following primers. The result is shown in Fig. 10.
PPAR-γ:
   (Forward: SEQ ID NO: 1)
      5'-CGAGCCCTGGCAAAGCATTTGTAT-3'
   (Reverse: SEQ ID NO: 2)
      5'-TGTCTTTCCTGTCAAGATCGCCCT-3'

As will be apparent from Fig. 10, expression amount of PPAR-γ mRNA in kidney significantly elevated to 133% for CS group, 141% for Si group and 136% for Sr group as compared with 0.56 (100%) for the control group and it was shown that the agent for prevention or treatment of the elevated blood glucose level according to the present invention promoted the oxidation/combustion of fat whereby the kidney was protected from the progress to nephropathy.

### (Quantification of expression of adiponectin mRNA in kidney)

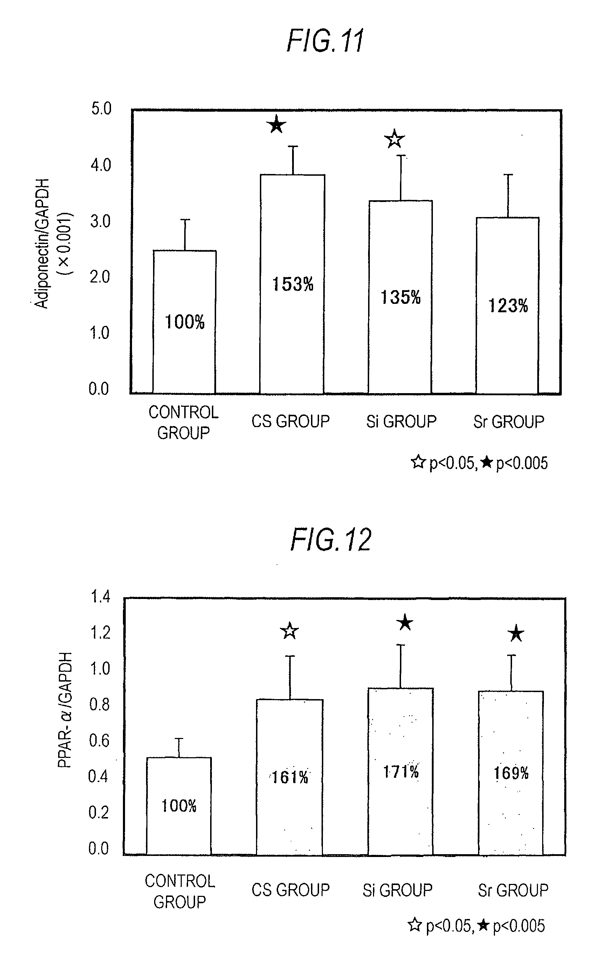

Expression amount of adiponectin mRNA in kidney was measured by the PCR using the following primers. The result is shown in Fig. 11.
Adiponectin:
   (Forward: SEQ ID NO: 3)
      5'-GCACTGGCAAGTTCTACTGCAACA-3'
   (Reverse: SEQ ID NO: 4)
      5'-AGAGAACGGCCTTGTCCTTCTTGA-3'

As will be apparent from Fig. 11, expression amount of adiponectin mRNA significantly elevated to 153% for CS group, 135% for Si group and 123% for Sr group as compared with 2.53 × 10⁻³ (100%) for the control group and it was shown that the agent for prevention or treatment of the elevated blood glucose level according to the present invention promoted the oxidation/combustion of fat whereby the kidney was protected from the progress to nephropathy.

### (Quantification of expression of PPAR-α mRNA in kidney)

Expression amount of PPAR-α mRNA in kidney was measured by the PCR using the following primers. The result is shown in Fig. 12.
PPAR-α:
   (Forward: SEQ ID NO: 5)
      5'-AAGAACCTGAGGAAGCCGTTCTGT-3'
   (Reverse: SEQ ID NO: 6)
      5'-GCAGCCACAAACAGGGAAATGTCA-3'

As will be apparent from Fig. 12, expression amount of PPAR-α mRNA significantly elevated to 160% for CS group, 171% for Si group and 169% for Sr group as compared with 0.532 (100%) for the control group and it was shown that the agent for prevention or treatment of the elevated blood glucose level according to the present invention promoted the oxidation/combustion of fat whereby the kidney was protected from the progress to nephropathy.

### Example 3

Genetic examination (2):
Aged patients suffering from diabetes have a higher risk of bone fracture than non-diabetic patients of the same ages (Schwatz A, Sellmeyer DE, Ensrud KE, et al., Older women with diabetes have an increased risk of fracture: a prospective study. J. clin. Endocrinol. Metab., 86, 32-38 (2001)) and, as shown above, there have been many reports for harmful effects to bone metabolism by antidiabetic thiazolidinedione derivatives (Rzonca SO, Suva LJ, Gaddy D, et al., Bone is a target for the antidiabetic compound rosigltazone. Endocrinology, 145, 401-406 (2004)). Therefore the action of the agent for prevention or treatment of the elevated blood glucose level according to the present invention on bone metabolism was tested.

After the mice in each group of Example 1 were sacrificed, RNA was extracted from bone marrow cells of femur according to the conventional method. cDNA was prepared from the RNA by a reverse transcription reaction and a quantitative determination of expression amount of mRNA related to the bone metabolism was conducted by means of a real-time quantitative PCR.

As a result, the expression amount of intra-nuclear receptor PPARγ which is a differentiation transcription factor to adipocytes upon differentiation of undifferentiated germ layer stem cells to either osteoblasts or adipocytes significantly lowered to a medium extent of 46% for CS group, 50% for Si group and 65% for Sr group as compared with 5.05 × 10⁻² (100%) for the control group whereby a favorable result for bone metabolism was achieved together with various other indexes.

### Industrial Applicability

The compound containing soluble silicon and/or strontium according to the present invention is able to significantly reduce the blood glucose level and insulin level *in vivo* due to the action of soluble silicon and/or strontium contained therein and it is useful for the prevention and the treatment of insulin-dependent type II diabetes.

It was reported that, when the soluble silicon as ³²Si with a half life of 150 years was given to healthy persons, about 50% of the ingested dose was absorbed, urinary elimination occurred by two-phase processes with half-lives of 2.7 and 11.3 hours in ³²Si, representing around 90% and 10%, respectively, of the total output, and the elimination of absorbed ³²Si was essentially completed after 48 hours (Popplewell JF, et al., J. Inorg. Biochem., 69, 177-180 (1998)) and, accordingly, it is judged to have a high safety without accumulation in the body.

Further, there is a report that, with regard to strontium, when a daily dose of 6.4 g of strontium lactate (1.7 g as Sr) was administered for three months to three years to 32 patients suffering from osteoporosis, improvement was achieved in 84% of the patients (Janes JM, McCaslin F., The effect of strontium lactate in the treatment of osteoporosis. Mayo. Clin. Proc., 43, 329-443 (1959)) and, therefore, it is judged to be with high safety.

As such, the agent for prevention or treatment of the elevated blood glucose level according to the present invention is able to be widely used as an agent of a new type for prevention or treatment of insulin-dependent type II diabetes and, particularly when it is used together with an antidiabetic agent comprising a thiazolidinedione derivative where tendency of lowering of bone mass is a problem, it is expected that the lowering of the bone mass is able to be prevented at the same time.

### Brief Description of the Drawings

[Fig. 1] Changes in blood glucose level during the feeding period.
[Fig. 2] Relation between the diets used and the blood glucose level at the end of feeding.
[Fig. 3] Relation between the diets used and the plasma insulin level at the end of feeding.
[Fig. 4] Relation between the diets used and the plasma leptin level at the end of feeding.
[Fig. 5] Relation between the diets used and the plasma adiponectin level at the end of feeding.
[Fig. 6] Relation between the diets used and the expression amount of PPAR-γ mRNA in pancreas.
[Fig. 7] Relation between the diets used and the expression amount of adiponectin mRNA in pancreas.
[Fig. 8] Relation between the diets used and the expression amount of PPAR-α mRNA in pancreas.
[Fig. 9] Relation between the diets used and the expression amount of insulin mRNA in pancreas.
[Fig. 10] Relation between the diets used and the expression amount of PPAR-γ mRNA in kidney.
[Fig. 11] Relation between the diets used and the expression amount of adiponectin mRNA in kidney.
[Fig. 12] Relation between the diets used and the expression amount of PPAR-α mRNA in kidney.

## Claims

1. An agent for prevention or treatment of the elevated blood glucose level, comprising a compound containing soluble silicon and/or strontium as an active ingredient.

2. The agent for prevention or treatment of the elevated blood glucose level according to claim 1, wherein the soluble silicon is compounded in an amount of 5 µg to 200 mg as a daily dose.

3. The agent for prevention or treatment of the elevated blood glucose level according to claim 1 or 2, wherein the compound containing the soluble silicon is metasilicic acid or silicic acid or is sodium, potassium, calcium or magnesium salt thereof.

4. The agent for prevention or treatment of the elevated blood glucose level according to claim 1, wherein the strontium is compounded in an amount of 1.5 mg to 1,500 mg as a daily dose.

5. The agent for prevention or treatment of the elevated blood glucose level according to claim 1 or 4, wherein the compound containing strontium is strontium chloride, strontium bromate, strontium bromide, strontium hydroxide, strontium oxide, strontium carbonate, strontium phosphate or strontium sulfate.

6. The agent for prevention or treatment of the elevated blood glucose level according to claim 1 or 4, wherein the compound containing strontium is strontium acetate, strontium lactate, strontium oxalate or a strontium-containing organic synthetic compound.

7. The agent for prevention or treatment of the elevated blood glucose level according to claim 1, wherein the compound containing soluble silicon and strontium is unburned coral calcium.
